# EUROPEAN PATENT SPECIFICATION

(11) **EP 1 484 066 B1**
(45) Date of publication and mention of the grant of the patent: **21.11.2012**
(21) Application number: 03705402.0
(22) Date of filing: 24.02.2003
(51) Int. Cl.: A61K 38/17, A61P 31/04, A61P 31/06

(54) **15k granulysin for the treatment of infections**
15k Granulysin zur Behandlung von Infektionen
15k granulysine pour le traitement d'infections

(30) Priority: 22.02.2002 JP 2002045865
(43) Date of publication of application: 08.12.2004
(73) Proprietor: National Hospital Organization, Tokyo, 152-8621 (JP); Okada, Masaji, Osaka 591-8555 (JP); Takamori, Yasushi, Tokyo 178-0061 (JP); Azbio Corp, Osaka 530-0043 (JP)
(72) Inventor: OKADA, Masaji, RC4-201, Nat. Kinki-Chuo Hospital, Sakai-shi, Osaka 591-8555 (JP); TAKAMORI, Yasushi, chome, Nerima-ku, Tokyo 178-0061 (JP); OGAWA, Kazuyuki, c/o General Laboratory BML, INC., Kawagoe-shi, Saitama 350-1101 (JP); NAGATA, Kinya, c/o General Laboratory, BML, INC., Kawagoe-shi, Saitama 350-1101 (JP)
(74) Representative: Gemmell, Peter Alan
(86) International application number: PCT/JP2003/001970
(87) International publication number: WO 2003/070268

(56) References cited:
- EP-A- 0 320 806
- WO-A1-99/22755
- KRENSKY A M: "Granulysin: a novel antimicrobial peptide of cytolytic T lymphocytes and natural killer cells." BIOCHEMICAL PHARMACOLOGY. 15 FEB 2000, vol. 59, no. 4, 15 February 2000 (2000-02-15), pages 317-320, XP002361930 ISSN: 0006-2952
- PENA SUSAN V. ET AL.: 'Processing, subcellular localization and function of 519(granulysin), a human late T cell activation molecule with homology to small, lytic, granule proteins' JOURNAL OF IMMUNOLOGY vol. 158, no. 6, 1997, pages 26680 - 2688, XP002920631
- MA LING LING ET AL.: 'Anticryptococcal activity of mitogen stimulated primary human CD8+ T cells mediated by granulysin' FASEB JOURNAL vol. 15, no. 5, 2001, page A1009, 774.20, 1.9-10, 15-16, XP002968884
- STENGER STEFFEN: 'An antimicrobial activity of cytolytic T cells mediated by granulysin' SCIENCE vol. 282, 1998, pages 121 - 125, XP002920630

## Description

### TECHNICAL FIELD

The present invention relates to the use of 15 kg granulys in in medecine, in particular for treating infectious diseases such as tuberculosis.

### BACKGROUND TECHNIQUE

In medical care, it goes without saying that the existence of a remedy for infectious diseases is indispensable. At present, a number of remedies for infections such as antibiotics and synthetic antibacterial agents are provided, and are used in medical care.

However, currently, an antibacterial agent which is mainly provided as a remedy for infectious diseases is in fact accompanied with an unavoidable problem of appearance of drug resistant bacteria. That is, sarcastic state continues in which by provision of a new antibacterial agent, a new drug resistant bacterium is produced.

For example, in tuberculosis occupying one of the highest number of patient with in single infectious disease, a tendency of recent increase becomes a worldwide problem. Further, the existence of a drug resistant bacterium having resistance to almost all antibiotics has been confirmed, and this problem is being revealed.

Recently, it has been revealed that a molecule called granulysin expressed in a Natural Killer cells (NK cell) or Cytotoxic T Lymphocytes (CTL) has direct ability to kill and injure bacteria such as Mycobacterium tuberculosis (Stenger, S.et al., Science 282, 121-125(1998)).

Granulysin is produced as a precursor of 15K and, thereafter, processed into 9K in a cytotoxic granule, and it is known that this 9K granulysin has antibacterial activity (Pena, S.V.et al., J.Immunol, 158, 2680-2688 (1997)). Further, a pathway is reported in which perforin which is the molecule derived from the same cytotoxic intragranule perforates a target cell, granulysin enters into a cell therethrough, and kills and injures an infecting bacterium in a cell (Stenger, S. et.al., Science 282, 121-125 (1998)).

Like this, it is thought that granulysin plays an important role in defense for infection. It is hardly thought that active type 9K granulysin makes a bacterium thereto acquire resistance, and application to a remedy for infectious diseases having different characteristic from an antibiotic is contemplated. In addition, recently, the existence of perforin pathway regarding removal of an intracellular infectious bacterium by 9K granulysin is suspected (David, H.C. et al., J.Immunol, 167, 2734-2742 (2001)). Moreover, cytotoxity is perceived in this molecule (Pena, S.V. et al., J.Immunol, 158, 2680-2688 (1997)) and, when this is administered as such, considerable side effect toxicity is feared.

A problem to be solved by the invention is to study granulysin in further detail, and provide means for using this as a remedy for infectious diseases.

### DISCLOSURE OF THE INVENTION

As a result; the present inventors found out a novel pathway wherein 15K granulysin is incorporated into a macrophage and, thereafter, activated to kill and injure bacteria phagocytosed into a macrophage.

That is, the present inventors found out that use of 15K granulysin showing no cytotoxicity in itself as an active ingredient makes it possible to provide an efficacious remedy for infections which has little side effect and to which bacteria can acquire little tolerance.

The present invention provides the use of an infection remedy of which the active ingredient is 15K granulysin (hereinafter also referred to as the present remedy).

In the present invention, 15K granulysin is a protein, having a molecular weight of 15,000 (15k), consisting of 145 amino acids which is localized in cytotoxic granule as described above. In a cytotoxic granule, a part of 15K granulysin is cut, and 15K granulysin is present as a protein having a molecular weight of 9,000 (9k) (Pena, S.V., et al., J.Immunol., 158, 2680-2688 (1997), Stenger, S., et al., Science, 282, 121-125 (1998)).

### BRIEF DESCRIPTION OF THE DRAWINGS

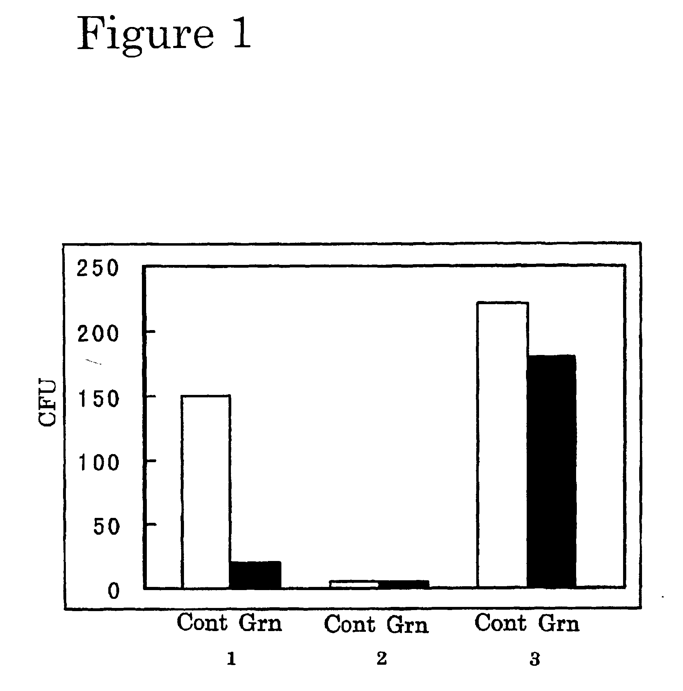

Fig. 1 is a view showing results of study on antibacterial effect of 15K granulysin on Mycobacterium tuberculosis.

### BEST MODE FOR CARRYING OUT THE INVENTION

Embodiments of the present invention will be explained below.

### A. Active ingredient of present remedy

15K granulysin used as an active ingredient of the present remedy can be obtained from a living body, but since 15K granulysin is a trace component in a living body, it is preferable to use a recombinant protein obtained by expressing a gene encoding 15K granulysin. Alternatively, it is also a suitable means to produce 15K granulysin in a living body, utilizing an intracorporeal expression vector for 15K granulysin in which a gene encoding 15K granulysin is inserted, as an active ingredient.

### (i) Recombinant protein of 15K granulysin

A sequence of a gene encoding 15K granulysin has been already reported (Jongstra, et al., J.Exp.Med, 165, 601), specifically, a gene having a nucleotide sequence represented by SEQ ID NO: 1 and a 15K granulysin protein having an amino acid sequence corresponding thereto. Based on this, a gene encoding 15K granulysin is effectively prepared, and this gene is expressed, whereby, a recombinant protein of 15K granulysin can be prepared.

Specifically, a gene amplification product of a gene encoding 15K granulysin is prepared using nucleotide chains complementary to both ends of a sequence of a gene encoding 15K granulysin as an amplification primer, by a gene amplification method such as a PCR method.

This is transduced into a suitable gene expression vector, and desired 15K granulysin can be obtained from a suitable host such as Escherichia coli, Bacillus subtilis, yeast and insect cell transformed with such the recombinant vector.

It is preferable that, as a gene expression vector used herein, a vector usually harboring a promoter and an enhancer at a region upstream of a gene to be expressed, and a transcription termination sequence at a region downstream of the gene is used.

In addition, expression of a 15K granulysin gene is not limited to a direct expression system. For example, a fused protein expression system utilizing a β-galactosidase gene, a glutathione-S-transferase gene or a thioredoxin gene may be used.

As a gene expression vector using Escherichia coli as a host, there are exemplified pQE, pGEX, pT7-7, pMAL, pTrxFus, pET, and pNT26CII. As a vector using Bacillus subtilis as a host, there are exemplified pPL608, pNC3, pSM23, and pKH80.

In addition, as a vector using yeast as a host, there are exemplified pGT5, pDB248X, pART1, pREP1, YEp13, YRp7, and YCp50.

In addition, as a vector using a mammal cell or an insect cell as a host, there are exemplified p91023, pCDM8, pcDL-SRα296, pBCMGSNeo, pSV2dhfr, pSVdhfr, pAc373, pAcYM1, pRc/CMV, pREP4, and pcDNAI.

These gene expression vectors can be selected depending on the purpose of expression of 15K granulysin. For example, when 15K granulysin is expressed, it is preferable to select a gene expression vector for which Escherichia coli, Bacillus subtilis or yeast can be selected as a host. When 15K granulysin is expressed even at a small amount so that it has assuredly activity, it is preferable to select a gene expression vector for which a mammal cell or an insect cell can be selected as a host.

In addition, it is possible to select the existing gene expression vector as described above, but a gene expression vector is appropriately produced depending on a purpose, and this may be of course used.

Transfection of the gene expression vector in which a 15K granulysin gene is inserted, into a host cell, and a transformation method therewith can be performed by the general method, for example, a calcium chloride method and an electroporation method in the case of Escherichia coli and Bacillus subtilis as a host cell, and by the means such as a calcium phosphate method, an electroporation method and a liposome method in the case of a mammal cell and an insect cell as a host.

By the culture of the thus obtained transformant according to the conventional method, desired 15K granulysin is accumulated.

Medium used in such the culture can be appropriately selected depending on a host. For example, in the case of Escherichia coli as a host, LB medium and TB medium can be appropriately used and, in the case of mammal cells as a host, RPMI1640 medium can be appropriately used.

Isolation and purification of 15K granulysin from a culture obtained by this culture can be performed according to the conventional method. For example, isolation and purification can be performed by subjecting to the culture to various treating operations utilizing physical and/or chemical nature of 15K granulysin.

Specifically, treatment with a protein precipitating agent, ultrafiltration, gel filtration, high performance liquid chromatography, centrifugation, electrophoresis, affinity chromatography using a specific antibody, and dialysis method can be used alone or by combining these methods.

As described above, 15K granulysin can be separated and purified.

By using 15K granulysin as an active ingredient in a remedy for infectious diseases, it is incorporated into a macrophage in blood, and activated therein, whereby, it kills and injures bacteria, viruses, and fungi inducing infections which have been phagocytosed by a macrophage, whereby, it becomes possible to treat infections due to these microoraganisms. As described above, unlike 9K granulysin, cytotoxicity is not perceived in 15K granulysin per se, and effects as a remedy for infectious diseases having little side effect due to administration are expected.

### (ii) Intracorporeal expression vector for 15K granulysin

An active ingredient of the present remedy in this form is a recombinant vector in which a gene encoding 15K granulysin used for expressing the aforementioned recombinant protein is inserted into an intracorporeal expression vector.

Examples of the intracorporeal expression vector are not limited to, but include an adenovirus vector and a retrovirus vector.

For a recombinant intracorporeal expression vector, for example, an expressible gene in which 15K granulysin is further transduced into a cosmid vector in which the aforementioned virus gene has been transduced, is incorporated and then, this cosmid vector and a parent virus DNA - TP which have been restriction enzyme-treated are transfected into 293 cells, whereby, homologous recombination occurs in the 293 cells, thus, a desired intracorporeal expression vector is produced.

### B. Form of present remedy

### (i) Present remedy using recombinant protein of 15K granulysin as active ingredient

In the first form of the present remedy, 15K granulysin is incorporated as an active ingredient and, at the same time, an appropriate pharmaceutical preparation carrier can be incorporated to prepare into a r form of a preparation composition (of course, only 15K granulysin is possible). As a pharmaceutical preparation carrier, for example, excipients and diluents such as fillers, bulking agents, binders, wetting agents, stabilizers, solubilizers, disintegrating agents and surface active agents which can be conventionally used as a pharmaceutical preparation carrier can be appropriately selected depending on the specific dosage form. A form of a preparation composition is not particularly limited as far as it is such a form that 15K granulysin can be effectively used in utilities of for treatment of infectious diseases. For example, even solid agents such as tablets, powders, granules and pills may be formulated into an injectable form such as solutions, suspensions and emulsions. By adding an appropriate carrier to 15K granulysin, a dried agent can be obtained which can be made to be liquid upon use.

A dose of the thus obtained present remedy can be appropriately selected depending on an administration method and an administration form of an agent, and symptom of a patient, being not particularly limited.

Such various forms of pharmaceutical preparations can be administered by an appropriate administration route depending on its form, for example, by intravenous, intramuscular, intraosseous, intra-articular, subcutaneous, intracutaneous or intraperitoneal administration in the case of an injectable form, or by oral or enteral administration in the case of a solid agent form.

### (ii) Present remedy using intracorporeal expression vector for 15K granulysin as an active ingredient.

By isolating and purifying the intracorporeal expression vector which can be prepared as described above, and administering this to a living body, 15K granulysin is produced in a living body, and the pharmacological effect of this 15K granulysin can be exerted.

A dosage form in this case is generally an injectable form, and the remedy can be administered by intravenous, intramuscular, intraosseous, intra-articular, subcutaneous, intracutaneous or intraperitoneal administration. A dose of such the present remedy can be appropriately selected depending on an administration method and an administration form of an agent, and symptom of a patient, being not limited. Generally, it is preferable to appropriately prepare an intracorporeal expression vector expressing 15K granulysin as an active ingredient, and administer this preparation at an appropriate amount once per day or by dividing into a few times per day.

### EXAMPLE

Examples of the present invention will be described below.

### [Test Example] Study of antibacterial effect on Mycobacterium tuberculosis when 15K granulysin and a macrophage are present jointly

### (1) Preparation of monoclonal antibody specific for 15K granulysin

A RNA was extracted from human peripheral blood lymphocyte which had been cultured in the presence of 100 to 200 unit/ml of IL-2 (2×10⁶ cells/ml of human peripheral blood lymphocytes were cultured at 37°C under 5% CO₂ incubator for 10 days in RPMI1640 medium containing 10% fetal bovine serum) by the conventional method, and a RT-PCR method (PCR primer1: SEQ ID NO:2, PCR primer 2:SEQ ID NO: 3) was performed using this RNA as a template, to synthesize a gene part containing a region encoding a full length protein of 15K granulysin (Jongstra et al., J. Exp.Med, 165, 601: part corresponding to amino acid sequence of SEQ ID NO:1) as the amplification product of a complementary DNA (cDNA). This cDNA encoding a full length protein of 15K granulysin was incorporated into pRc/CMV or pcDL-SRα296 which is a mammal expression vector, the resulting recombinant vector was dissolved in a physiological saline, and a mouse was immunized with the solution subcutaneously or intracutaneously. After 4 to 5 times immunization at an interval of 1 to 2 weeks, spleen cells obtained from mouse for which increase in an antibody titer was perceived by an indirect fluorescent antibody method (performed according to the method described later) was cell-fused according to the conventional method, and a hybridoma producing an antibody which specifically binds to granulysin was retrieved again with an indirect fluorescent antibody method. That is, the cells were transfected with the aforementioned gene encoding 15K granulysin, cells (Cos7) in which the gene was expressed was fixed with 4% paraformaldehyde, membranes were solubilized with 0.5% Tween 20, the culture supernatant of hybridoma cells was added thereto to react with an antibody, this was reacted with a fluorescently labeled anti-mouse IgG antibody, and fluorescence was detected, whereby, a hybridoma producing an antibody which specifically binds to granulysin was screened. As a result, 9 hybridomas producing an antibody which specifically binds to granulysin were obtained. Using the culture supernatant of each of the resulting hybridomas, ammonium sulfate precipitation and purification by Protein G column were performed to prepare two kinds of monoclonal antibodies to 15K granulysin. Hereinafter, this is referred to monoclonal antibody RF10 (which binds to 15K granulysin, but does not bind to 9K granulysin) or monoclonal antibody RC8 (which binds to both 15K granulysin and 9K granulysin).

### (2) Preparation of polyclonal antibody to 15K granulysin

A rabbit was immunized with a conjugate of a partial amino acid sequence (29 amino acids) of granulysin (J. Exp. Med. 165:601-614 (1987), J. Exp.Med., 172:1159-1163 (1990)): Arg Thr Gly Arg Ser Arg Trp Arg Asp Val Cys Arg Asn Phe Met Arg Arg Tyr Gln Ser Arg Val Thr Gln Gly Leu Val Ala Gly (N5-1: SEQ ID NO: 4) and limpet hemocyanin according to the conventional method, to obtain anti-serum. The resulting antiserum was purified by ammonium sulfate precipitation and Protein G column, and further purified by affinity chromatography using a column bound with the aforementioned synthetic peptide (N5-1), to prepare a polyclonal antibody (anti-N5-1 antibody) to granulysin.

### (3) Preparation of granulysin-containing culture supernatant

A gene recombinant vector was produced, in which, among a nucleotide sequence of SEQ ID NO:1, a nucleotide chain of a nucleotide sequence encoding a part corresponding to 15K granulysin protein was incorporated into pFLAG-CMV vector (manufactured by Sigma) according to the conventional method. As a control, the pFLAG-CMV vector in which recombination with a gene was not performed, was used. Each of these gene recombinant vectors was transfected into a Cos7 cell, and this was cultured at 37°C under 5% CO₂ for 72 hours in DMEM medium (manufactured by Gibco). After completion of culture, the culture was centrifuged (2500 rpm·20 min·4°C) to obtain the culture supernatant.

Regarding each of the culture supernatants, the proteins were separated by SDS-PAGE. After the protein was transferred to a nylon membrane from a gel of electrophoresed SDS-PAGE, the membrane was blocked with blocking solution (1% skim milk/washing solution), to this was bound monoclonal antibody RF10 specific for 15K granulysin, and enzyme-labeled anti-mouse antibody and color developing substrate were acted on these, to develop a bond. As a result, in the supernatant obtained by expressing a gene encoding 15K granulysin protein, a band exhibiting a molecular weight of 15K appeared. However, in the control, this bond was not perceived. In addition, when the similar test was performed using a polyclonal antibody N5-1 binding to 15K and 9K granulysins, a band showing 15K was perceived, but a band showing 9K was not perceived.

From this result, it was revealed that 15K granulysin was specifically present in the aforementioned granulysin culture supernatant, but granulysin was not present in control culture supernatant.

### (4) Study of antibacterial effect

Lymphocyte separated from human blood was suspended in a culture (RPMI1640, 10% human serum) to around 2×10⁷ cells in a plastic culture plate (24 wells/plate) per 1ml of medium according to the conventional method, each 1ml of this was dispensed into each well, allowed to stand at 37°C for 24 hours, lymphocyte was adhered to a plate surface wall and, in this adherent macrophage, antibacterial effect of 15K granulysin was studied.

Then, 1 ml of RPMI1640 (10% human serum) was added to a well, Mycobacterium tuberculosis (M.tuberculosis) (H37Rv, 1×10⁵ to 1×10⁶ cfu) was static-cultured at 37°C for 4 to 12 hours under 5% CO₂, to infect macrophages with M.tuberculosis. After completion of infection, each 1ml of supernatant containing 15K granulysin (Grn supernatant) and supernatant in the absence of 15K granulysin (Cont supernatant) was added to a well, and static culture was performed 2 to 12 hours under the same conditions.

After completion of culture, the culturing supernatant was removed, the adhered cells in a plate were washed with PBS three times, M.tuberculosis in a cell was extracted with total 5 ml of 1% aqueous saponin solution, this extract was diluted, seeded on a plate agar medium [7H11 medium (manufactured by Gibco)], this was static-cultured at 37°C for 14 days, and the number of colonies was counted.

The results are shown in Fig. 1.

In Fig. 1, Cont denotes control culturing supernatant, and Grn denotes granulysin culture supernatant. A coordinate axis denotes the number of colonies. 1 on an abscissa axis denotes the results obtained by contacting macrophages, M.tuberculosis and the culture supernatant, as described above. 2 denotes the result obtained by static-culturing each culture supernatant and M.tuberculosis in the absence of macrophages as in 1, washing this, static-culturing this in a 7H11 plate agar medium, and influence of M.tuberculosis on non-specific adsorption in a plate was confirmed. 3 denotes the result obtained by static- culture each 1ml of the culture supernatant and M.tuberculosis at 37°C for 2 hours in the aforementioned culture plate, and static-culture this in a 7H11 plate agar medium.

As a result, it was revealed that 15K granulysin has antibacterial effect on M.tuberculosis by intervention of macrophages. When macrophages did not intervene, antibacterial effect of 15K granulysin on M.tuberculosis was not perceived.

By the aforementioned Test Example, antibacterial activity was perceived in 15K granulysin, and it was revealed that 15K granulysin can be used as an active ingredient of a remedy for infections.

It has been reported that, when 15K granulysin was transfected into cells such as Cos7 cells, HeLa and PC12, 15K granulysin was detected in the culture supernatant, but damage was not perceived in the cells. When 9K granulysin was used in place of 15K granulysin in the presence of perforin, cytotoxic activity or apoptosis is induced (Pena, S. V. et al, J. Immunol., 158, 2680-2688(1997)).

### INDUSTRIAL APPLICABILITY

According to the present invention, there is provided the use of a remedy for infections containing 15K granulysin as an active ingredient, which has no perceived side effect, and is effective.

## Claims

1. 15k granulysinfor use as a medicament.

2. 15k granulysin for use in the treatment of infection or infectious diseases.

3. 15 k granulysin for use according to Claim 2, wherein the infection or infectious disease is bacterial, viral or fungal.

4. 15k granulysin for use according to Claim 2 or Claim 3, wherein the infection or infectious disease is bacterial.

5. 15k granulysin for use according to any of Claims 2 to 4, wherein the infection or infectious disease is caused by *Mycobacterium tuberculosis.*

6. 15k granulysin for use according to any of Claims 2 to 5, wherein the bacteria, viruses or fungi have been phagocytosed by a macrophage.

7. 15k granulysin for use according to any preceding claim, wherein the 15K granulysin is a recombinant protein.

8. 15k granulysin for use according to any preceding claim, wherein the 15k granulysin is in the form of an intracorporeal expression vector for 15k granulysin, in which a gene encoding 15k granulysin is incorporated.

9. 15k granulysin for use according to any preceding claim, wherein the 15k granulysin forms part of a pharmaceutical composition and wherein the pharmaceutical composition further comprises a pharmaceutical carrier.

10. 15k granulysin for use according to Claim 9, when dependent on any of Claims 1 to 7, wherein the pharmaceutical composition is in solid dosage form.

11. 15k granulysin for use according to Claim 9, wherein the pharmaceutical composition is in injectable form.

## Patentansprüche

1. 15k Granulysin zur Verwendung als Medikament.

2. 15k Granulysin zur Verwendung bei der Behandlung von Infektionen oder Infektionskrankheiten.

3. 15k Granulysin zur Verwendung nach Anspruch 2, wobei die Infektion oder Infektionskrankheit bakteriell, viral oder eine Mykose ist.

4. 15k Granulysin zur Verwendung nach Anspruch 2 oder Anspruch 3, wobei die Infektion oder die Infektionskrankheit bakteriell ist.

5. 15k Granulysin zur Verwendung nach einem der Ansprüche 2 bis 4, wobei die Infektion oder die Infektionskrankheit durch Mycobacterium Tuberculosis verursacht wird.

6. 15k Granulysin zur Verwendung nach einem der Ansprüche 2 bis 5, wobei die Bakterien, Viren oder Pilze durch einen Makrophagen phagozytiert wurden.

7. 15k Granulysin zur Verwendung nach einem der vorherigen Ansprüche, wobei das 15k Granulysin ein rekombinantes Protein ist.

8. 15k Granulysin zur Verwendung nach einem der vorherigen Ansprüche, wobei das 15k Granulysin als intrakorporealer Expressionsvektor für 15k Granulysin vorliegt, in dem ein Gen, das 15k Granulysin kodiert, enthalten ist.

9. 15k Granulysin zur Verwendung nach einem der vorherigen Ansprüche, wobei das 15k Granulysin Bestandteil einer pharmazeutischen Zusammensetzung ist und wobei die pharmazeutische Zusammensetzung ferner einen pharmazeutischen Träger umfasst.

10. 15k Granulysin zur Verwendung nach Anspruch 9, sofern abhängig von einem der Ansprüche 1 bis 7, wobei die pharmazeutische Zusammensetzung in einer festen Dosierform vorliegt.

11. 15k Granulysin zur Verwendung nach Anspruch 9, wobei die pharmazeutische Zusammensetzung in injizierbarer Form vorliegt.

## Revendications

1. Granulysine 15k à utiliser comme médicament.

2. Granulysine 15k à utiliser dans le traitement d'une infection ou de maladies infectieuses.

3. Granulysine 15k à utiliser selon la revendication 2, où l'infection ou la maladie infectieuse est bactérienne, virale ou fongique.

4. Granulysine 15k à utiliser selon la revendication 2 ou la revendication 3, où l'infection ou la maladie infectieuse est bactérienne.

5. Granulysine 15k à utiliser selon l'une quelconque des revendications 2 à 4, où l'infection ou la maladie infectieuse est provoquée par *Mycobacterium tuberculosis.*

6. Granulysine 15k à utiliser selon l'une quelconque des revendications 2 à 5, où les bactéries, les virus ou les champignons ont été phagocytés par un macrophage.

7. Granulysine 15k à utiliser selon l'une quelconque des revendications précédentes, où la granulysine 15k est une protéine recombinante.

8. Granulysine 15k à utiliser selon l'une quelconque des revendications précédentes, où la granulysine 15k est sous forme d'un vecteur d'expression intracorporel pour la granulysine 15k, dans lequel est incorporé un gène codant pour la granulysine 15k.

9. Granulysine 15k à utiliser selon l'une quelconque des revendications précédentes, où la granulysine 15k fait partie d'une composition pharmaceutique et où la composition pharmaceutique comprend en outre un support pharmaceutique.

10. Granulysine 15k à utiliser selon la revendication 9 lorsqu'elle dépend de l'une quelconque des revendications 1 à 7, où la composition pharmaceutique est sous une forme galénique solide.

11. Granulysine 15k à utiliser selon la revendication 9, où la composition pharmaceutique est sous une forme injectable.


### SEQUENCE LISTING

<110> BML, INC. NATIONAL KINKI-CHUO HOSPITAL FOR CHEST DISEASES Okada, Masaji
<120> Remedy for Infectious Diseases
<130> PBM67/PCT
<150> JP 2002-45865
   <151> 2002-02-22
<160> 4
<170> PatentIn version 3.1
<210> 1
   <211> 745
   <212> DNA
   <213> Homo sapiens
<220>
   <223> Inventor: Okada, Masaji Inventor: Takamori, Yasushi Inventor: Ogawa, Kazuyuki Inventor: Nagata, Kinya
<220>
   <221> CDS
   <222> (129).. (563)
   <223>
<400> 1
   gtatctgtgg taaacccagt gacacggggg agatgacata caaaaagggc aggacctgag      60
   aaagattaag ctgcaggctc cctgcccata aaacagggtg tgaaaggcat ctcagcggct     120
   gccccacc atg gct acc tgg gcc ctc ctg ctc ctt gca gcc atg ctc ctg      170
            Met Ala Thr Trp Ala Leu Leu Leu Leu Ala Ala Met Leu Leu
             1               5                  10
   ggc aac cca ggt ctg gtc ttc tct cgt ctg agc cct gag tac tac gac      218
   Gly Asn Pro Gly Leu Val Phe Ser Arg Leu Ser Pro Glu Tyr Tyr Asp
    15                 20                  25                  30
   ctg gca aga gcc cac ctg cgt gat gag gag aaa tcc tgc ccg tgc ctg      266
   Leu Ala Arg Ala His Leu Arg Asp Glu Glu Lys Ser Cys Pro Cys Leu
                   35                  40                  45
   gcc cag gag ggc ccc cag ggt gac ctg ttg acc aaa aca cag gag ctg      314
   Ala Gln Glu Gly Pro Gln Gly Asp Leu Leu Thr Lys Thr Gln Glu Leu
               50                  55                  60
   ggc cgt gac tac agg acc tgt ctg acg ata gtc caa aaa ctg aag aag      362
   Gly Arg Asp Tyr Arg Thr Cys Leu Thr Ile Val Gln Lys Leu Lys Lys
           65                  70                  75
   atg gtg gat aag ccc acc cag aga agt gtt tcc aat gct gcg acc cgg      410
   Met Val Asp Lys Pro Thr Gln Arg Ser Val Ser Asn Ala Ala Thr Arg
       80                  85                  90
   gtg tgt agg acg ggg agg tca cga tgg cgc gac gtc tgc aga aat ttc      458
   Val Cys Arg Thr Gly Arg Ser Arg Trp Arg Asp Val Cys Arg Asn Phe
    95                 100                 105                 110
   atg agg agg tat cag tct aga gtt acc cag ggc ctc gtg gcc gga gaa      506
   Met Arg Arg Tyr Gln Ser Arg Val Thr Gln Gly Leu Val Ala Gly Glu
                   115                 120                 125
   act gcc cag cag atc tgt gag gac ctc agg ttg tgt ata cct tct aca      554
   Thr Ala Gln Gln Ile Cys Glu Asp Leu Arg Leu Cys Ile Pro Ser Thr
               130                 135                 140
   ggt ccc ctc tgagccctct caccttgtcc tgtggaagaa gcacaggctc              603
   Gly Pro Leu
           145
   ctgtcctcag atcccgggaa cctcagcaac ctctgccggc tcctcgcttc ctcgatccag    663
   aatccactct ccagtctccc tcccctgact ccctctgctg tcctcccctc tcacgagaat    723
   aaagtgtcaa gcaagaaaaa aa                                             745
<210> 2
   <211> 24
   <212> DNA
   <213> Homo sapiens
<400> 2
   catctcagcg gctgccccac catg 24
<210> 3
   <211> 27
   <212> DNA
   <213> Homo sapiens
<400> 3
   tgtatacctt ctacaggtcc cctctga 27
<210> 4
   <211> 29
   <212> PRT
   <213> Homo sapiens
<400> 4
   Arg Thr Gly Arg Ser Arg Trp Arg Asp Val Cys Arg Asn Phe Met Arg
   1               5                   10                  15
   Arg Tyr Gln Ser Arg Val Thr Gln Gly Leu Val Ala Gly
               20                  25

### SEQUENCE LISTING

<110> BML, INC. NATIONAL KINKI-CHUO HOSPITAL FOR CHEST DISEASES Okada, Masaji
<120> Remedy for Infectious Diseases
<130> PBM67/PCT
<150> JP 2002-45865
   <151> 2002-02-22
<160> 4
<170> PatentIn version 3.1
<210> 1
   <211> 745
   <212> DNA
   <213> Homo sapiens
<220>
   <223> Inventor: Okada, Masaji Inventor: Takamori, Yasushi Inventor: Ogawa, Kazuyuki Inventor: Nagata, Kinya
<220>
   <221> CDS
   <222> (129) .. (563)
   <223>
<400> 1
   gtatctgtgg taaacccagt gacacggggg agatgacata caaaaagggc aggacctgag 60
   aaagattaag ctgcaggctc cctgcccata aaacagggtg tgaaaggcat ctcagcggct 120
   gccccacc atg gct acc tgg gcc ctc ctg ctc ctt gca gcc atg ctc ctg 170
   Met Ala Thr Trp Ala Leu Leu Leu Leu Ala Ala Met Leu Leu
   1 5 10
   ggc aac cca ggt ctg gtc ttc tct cgt ctg agc cct gag tac tac gac
   218
   Gly Asn Pro Gly Leu Val Phe Ser Arg Leu Ser Pro Glu Tyr Tyr Asp
   15 20 25 30
   ctg gca aga gcc cac ctg cgt gat gag gag aaa tcc tgc ccg tgc ctg
   266
   Leu Ala Arg Ala His Leu Arg Asp Glu Glu Lys Ser Cys Pro Cys Leu
   35 40 45
   gcc cag gag ggc ccc cag ggt gac ctg ttg acc aaa aca cag gag ctg
   314
   Ala Gln Glu Gly Pro Gln Gly Asp Leu Leu Thr Lys Thr Gln Glu Leu
   50 55 60
   ggc cgt gac tac agg acc tgt ctg acg ata gtc caa aaa ctg aag aag
   362
   Gly Arg Asp Tyr Arg Thr Cys Leu Thr Ile Val Gln Lys Leu Lys Lys
   65 70 75
   atg gtg gat aag ccc acc cag aga agt gtt tcc aat gct gcg acc cgg
   410
   Met Val Asp Lys Pro Thr Gln Arg Ser Val Ser Asn Ala Ala Thr Arg
   80 85 90
   gtg tgt agg acg ggg agg tca cga tgg cgc gac gtc tgc aga aat ttc
   458
   Val Cys Arg Thr Gly Arg Ser Arg Trp Arg Asp Val Cys Arg Asn Phe
   95 100 105 110
   atg agg agg tat cag tct aga gtt acc cag ggc ctc gtg gcc gga gaa
   506
   Met Arg Arg Tyr Gln Ser Arg Val Thr Gln Gly Leu Val Ala Gly Glu
   115 120 125
   act gcc cag cag atc tgt gag gac ctc agg ttg tgt ata cct tct aca 554
   Thr Ala Gln Gln Ile Cys Glu Asp Leu Arg Leu Cys Ile Pro Ser Thr
   130 135 140
   ggt ccc ctc tgagccctct caccttgtcc tgtggaagaa gcacaggctc 603
   Gly Pro Leu
   145
   ctgtcctcag atcccgggaa cctcagcaac ctctgccggc tcctcgcttc ctcgatccag 663
   aatccactct ccagtctccc tcccctgact ccctctgctg tcctcccctc tcacgagaat 723
   aaagtgtcaa gcaagaaaaa aa 745
<210> 2
   <211> 24
   <212> DNA
   <213> Homo sapiens
<400> 2
   catctcagcg gctgccccac catg 24
<210> 3
   <211> 27
   <212> DNA
   <213> Homo sapiens
<400> 3
   tgtatacctt ctacaggtcc cctctga 27
<210> 4
   <211> 29
   <212> PRT
   <213> Homo sapiens
<400> 4
   Arg Thr Gly Arg Ser Arg Trp Arg Asp Val Cys Arg Asn Phe Met Arg
   1 5 10 15
   Arg Tyr Gln Ser Arg Val Thr Gln Gly Leu Val Ala Gly
   20 25